# EUROPEAN PATENT APPLICATION

(11) **EP 1 886 623 A2**
(43) Date of publication of application: **13.02.2008**
(21) Application number: 07110585.2
(22) Date of filing: 19.06.2007
(51) Int. Cl.: A61B 5/00

(54) **System for detecting allergic reactions resulting from a chemical substance given to a patient**

(30) Priority: 30.06.2006 US 480344
(71) Applicant: General Electric Company, Schenectady, NY 12345 (US)
(72) Inventor: Uutela, Kimmo, 00100 Helsinki (FI); Rantala, Borje, 00670 Helsinki (FI)
(74) Representative: Bedford, Grant Richard

(57) **Abstract**

System for detecting allergic reactions resulting from a chemical substance given to a patient. The system comprises a workstation (2) configured to carry out desired proceedings and physiological measurements of the patient (1), a detection arrangement (3) for detecting the chemical substance given to the patient (1), a database (7) having information of allergic symptoms, and a warning/information display (8). The database (7) is configured to operate with the workstation (2) to obtain results of the physiological measurements carried out, and is further configured to operate with the detection arrangement (3) for detecting the chemical substance given to the patient (1) and the warning/information display (8) in order to generate a warning of allergic reactions from the basis of the information obtained from the physiological measurements and the chemical substance given to the patient (1).

## Description

The invention is related generally to a system for detecting allergic reactions resulting from a chemical substance given to a patient.

Different allergic reactions can lead to very difficult situations in hospital circumstances since many drugs may produce severe allergic reactions in some patients. These incidents are however generally taken rare and the symptoms may be nonspecific and therefore many clinicians may have troubles to identify said incidents. These troubles may even lead to lethal situations if the clinicians are not able to identify the origins of the incidents. The term allergic reactions used here must be understood widely, i.e. so that for example hypersensitive reactions are also included in the term used.

Anaesthesiology is one of specific fields in which patient receives a large number of drugs and other substances over a relatively short period of time. Any drug administered during the perioperative period can produce potentially a life-threatening allergic reaction.

As an other example of specific fields in which allergic reactions and problems connected to said reactions often occur critical care treatments can be mentioned.

The most common causes of severe allergic reactions (anaphylaxis) from drugs during anaesthesia are neuromuscular blocking agents (NMBAs) (58.2%), latex (16.7%), and antibiotics (15.1%). Rocuronium and succinylcholine were the most frequently incriminated NMBAs. These matters have been described in an article of Paul Michael Mertes, M.D., Ph.D., et al. (Anesthesiology, V 99, No 3, Sep 2003).

Deaths reported recently involved iodine contrast agents, rocuronium, protease inhibitors such as gabexate mesylate, aprotinin, cisplatin, nonstereoidal anti-inflammatories (NSAID; diclofenac, ketorolac), clindamycin, hydrocodone, methylpredisolone, dexrazoxane, and agens used for investigations such as patent blue, fluorescein, etc. All routes of administration are potentially lethal: intra-articular, intra-uterine, intra-lymphatic, inhalation, rectal, topical skin application, and even prick-tests. These matters have been described in an article of D.A. Moneret-Vautrin et al. (Allergy 2005: 60 443-451).

In contrast to food anaphylaxis, drug anaphylaxis is characterized by a high frequency of cardiovascular collapse with rapid onset within minutes, especially in older patients (Moneret-Vautrin et. al.).

Clinical features of anaphylactic reactions during anaesthesia include cardiovascular symptoms (74.7%), including arterial hypotension, cardiovascular collapse, bradycardia and cardiac arrest, broncospasm (39.8%), cutaneous symptoms (71.9%), and angioedema (12.3%) (Mertes et al.).

The matters described above clearly indicate that allergic reactions create a difficult problem in hospitals as regards drug administration. It is however also important to realize that severe allergic reactions does not relate only to drugs but also other chemical substances delivered to a patient, for example food, may lead to severe allergic reactions.

Although allergic reactions may in some circumstances lead to difficult situations there exists no proper solution for eliminating the disadvantages related to allergic reactions. As examples of the solutions of the prior art U.S. Patent specification No. 5 897 506 and U.S. Patent Application Publication No. 2004/0193325 A1 can be mentioned.

One object of the present invention is to obtain a system with which the problems related to detecting severe allergic reactions resulting from a chemical substance given to a patient can be eliminated or reduced. This object is obtained with various embodiments of the present invention. The system according to various embodiments of the invention is characterized in that the system comprises a workstation configured to carry out physiological measurements of the patient, a detection arrangement for detecting the chemical substance given to the patient, a database having information of allergic symptoms, and a warning/information display, the database being configured to operate with the workstation to obtain results of the physiological measurements carried out, and being further configured to operate with the detection arrangement for detecting the chemical substance given to the patient and the warning/information display in order to generate a warning of allergic reactions from the basis of the information obtained from the physiological measurements and the chemical substance given to the patient.

In the following various embodiments of the invention will be described in more detail by means of the study carried out with the drawing enclosed in which drawings:
Figure 1 shows principally one embodiment of the invention and
Figure 2 is a block diagram showing the operating principle of the system of various embodiments of the invention.

As described earlier a typical situation in which severe allergic reactions may occur relates to anaesthesiology, i.e. a patient is connected to a anaesthetic workstation and a drug or several drugs related to the proceedings to carried out is given to the patient, and the drugs given generate a severe allergic reaction. The invention will be described in the following in connection with an anaesthesia station, but it must be understood that the invention is by no means restricted to anaesthesiology.

The basis of the invention is a combination of drug detection system with for example anaesthesia workstation. This provides an easy way of recording drugs given to the patient. The system can also check and warn for drug interactions, known allergies of the patient, and provide instant access to drug-related information.

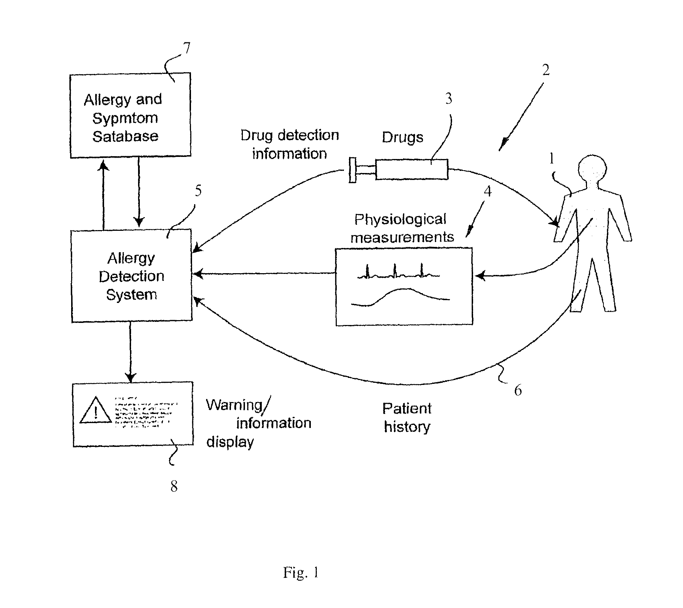

Figure 1 shows principally the system descried above. Reference number 1 shows the patient. Reference number 2 shows generally the workstation, for example anaesthesia workstation, used to carry out the proceedings desired. Reference number 3 shows generally a detection arrangement for detecting the chemical substance given to the patient 1. The arrangement detects what chemical substance and possible how much said chemical substance is given to the patient 1. The detection arrangement 3 can also inform when the chemical substance is given to the patient because timing is also an important feature as regards at least some severe allergic reactions. In other words it is possible within the spirit of the invention that the system collects information also on the time of giving the chemical substance and applies the information that the allergic reactions of a specific substance occur typically within a predefined time window after the substance is given. As an example of the chemical substances drugs can be mentioned. Barcodes, RFID tags, and user input can be used for identification of the chemical substances given to the patient.

Reference number 4 shows principally the arrangements for carrying out physiological measurements. For example in anaesthesia workstations the patient 1 in monitored by measuring for example blood pressure, ECG, pulse oximetry, air flows and pressures etc. As described earlier clinical features of anaphylactic and anaphylactoid reactions during anaesthesia comprise for example cardiovascular symptoms, bronchospasm and angioedema. Cardiovascular symptoms can be detected from the standard physiological patient monitoring including blood pressure measurement, ECG, and pulse oximetry. In ventilated patient, the narrowing of the airways related to the bronchospasm can be detected by changes in the air flow and airway pressures. In unventilated patients the condition is likely to change respiration rate and oxygenation. In severe angioedema, stridor of the airway occurs with gasping inspiratory breath sounds and decreasing oxygen levels.

The information obtained from physiological measurements and chemical substances fed to the patient are transmitted to a head unit 5 of the allergy detection system as shown by arrows in Figure 1.

Reference number 6 shows principally the arrangement for feeding information concerning known allergies of the patient 1 to the head unit 5 of the system. This feature is not compulsory in the invention but can be used if needed. The feeding of information can be manual or automatic, using, for example patient information databases. The system can then detect whether the patient has previously been given similar substances, and whether there have been previous allergic reactions. Often allergies of the patients may be correlated; if patient is known to have a certain allergy, they are more likely to have allergies also to certain other substances. In this way, allergy information could be used to quantify the likelihood of allergic reactions even if the patient has not previously received the same substance. The arrangement 6 can be for example a user input or a reader by which the information needed can be read directly from a patient's information card. As said earlier the arrangement 6 can also be in connection to the hospital's patient database.

Reference number 7 shows principally an allergy and symptom database in which great variety of allergy and symptom information and information concerning measured physiological reactions created by different chemical substances has been stored beforehand.

Reference number 8 shows a warning/information display. The warning/information display may comprise arrangements for generating a visible and/or audible warning signal and also further a display for showing information concerning the allergic reaction in question.

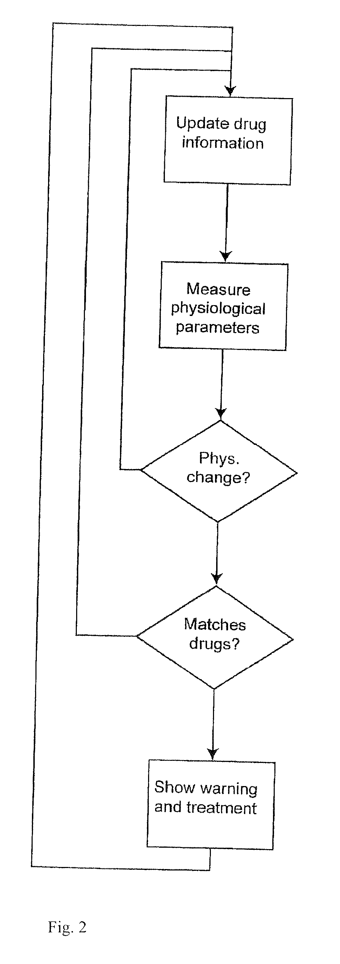

The idea in the invention is that the database 7 is configured to operate with the workstation 2 to obtain results of the physiological measurements carried out and also further configured to operate with the detection arrangement 3 for detecting the chemical substance given to the patient and eventually timing of the given chemical substance and the device 6 for feeding known allergies of the patient, and the warning/information display 8. Operation between the database 7 and workstation 3, the device 6 for feeding the known allergies of the patient and the warning/information display 8 is obtained by connecting the elements mentioned above to the head unit 5 of the system as shown in Figure 1. The connections are arranged in order to generate a warning of allergic reactions from the basis of the information obtained from the physiological measurements, the chemical substance given to the patient and also in some embodiments from the basis of timing of the drugs given and the known allergies of the patient fed to the system. The operation of the system is described principally in Figure 2. According to one aspect of the invention the information obtained as shown in Figure 2 is fed to the system and compared to the information stored earlier in the database, and the warning is generated if needed. The decision whether to show the warning, based on the different information sources, can be done with any of the methods used in the field, for example by using fuzzy logic or bayesian analysis.

As shown in Figure 2 the database 7 can also be updated when needed and when new information is available. The system monitors physical changes and compares with the other information, i.e. the drug information obtained etc., the results measured to the information stored beforehand into the database 7. If the comparison carried out matches with the information stored a warning is displayed.

Various embodiments of the invention are described above in connection with anaesthesia workstation. The invention is however not restricted to anaesthesia. The invention can be used also in connection with other workstations and arrangements for carrying out other purposes. Critical care monitors are another example in which the invention can be used.

As told above the chemical substance generating severe allergic reaction is very often a drug or several drugs in combination. The system of various embodiments of the invention may also detect the interactions of two or more chemical substances and physiological changes resulted from the interactions. Said physiological changes may be according to the invention detected and measured and may be included into information from basis of which the warning of allergic reactions is generated. For example arrangement 4 in the workstation 2 can be arranged to detect the physiological changes mentioned above.

The system of various embodiments of the invention could also detect and measure dosage data of the chemical substance given to the patient and physiological changes resulted from the dosage. Over and under dosing of a drug can be harmful or even dangerous to the patient in certain circumstances. The information described above may be detected and measured according to the spirit of the invention and may be included into information from the basis of which the warning of allergic reactions is generated. For example the detection arrangement 3 can be provided with means to detect and measure the dosage data described above. Physiological changes resulted from the dosage can measured by using the workstation 2.

The present invention is not restricted to drugs although the invention is described above in connection with drugs. The chemical substance generating severe allergic reactions can be any chemical substance, for example food including drinks etc.

## Claims

1. A system for detecting allergic reactions resulting from a chemical substance given to a patient, **characterized in that** the system comprises:
a workstation (2) configured to carry out desired physiological measurements of the patient (1),
a detection arrangement (3) for detecting the chemical substance given to the patient (1),
a database (7) having information of allergic symptoms, and
a warning/information display (8),
the database (7) being configured to operate with the workstation (2) to obtain results of the physiological measurements carried out, and being further configured to operate with the detection arrangement (3) for detecting the chemical substance given to the patient (1) and the warning/information display (8) in order to generate a warning of allergic reactions from the basis of the information obtained from the physiological measurements and the chemical substance given to the patient (1).

2. The system of claim 1, **characterized in that** the chemical substance is a drug.

3. The system of any preceding claim, **characterized in that** the chemical substance is food.

4. The system of any preceding claim, **characterized in that** the workstation (2) is an anaesthesia workstation.

5. The system of any preceding claim, **characterized in that** the workstation (2) is a critical care monitor.

6. The system of any preceding claim, **characterized in that** dosage data of the chemical substance given to the patient (1) and physiological changes resulted from the dosage are detected and measured and are included into information from the basis of which the warning of allergic reactions is generated.

7. The system of any preceding claim, **characterized in that** the interactions of two or more chemical substances and physiological changes resulted from the interactions are detected and measured and are included into information from basis of which the warning of allergic reactions is generated.

8. The system of any preceding claim, wherein the system further comprises a device (6) for feeding known allergies of the patient (1) to the system.

9. The system of any preceding claim, wherein the detection arrangement (3) also detects timing of the chemical substance given to the patient (1).
